(19) Europäisches Patentamt
European Patent Office
Office européen des brevets

(11) Veröffentlichungsnummer: **0 006 146**
**B1**

(12) # EUROPÄISCHE PATENTSCHRIFT

(45) Veröffentlichungstag der Patentschrift: **15.09.82**

(51) Int. Cl.³: **C 07 D 501/34,** A 61 K 31/545 // C07D277/24, C07D277/28, C07D277/32

(21) Anmeldenummer: **79101586.0**

(22) Anmeldetag: **23.05.79**

(54) **Cephemderivate, Verfahren zu ihrer Herstellung und pharmazeutische Präparate auf Basis dieser Verbindungen.**

(30) Priorität: **26.05.78 DE 2822860**

(43) Veröffentlichungstag der Anmeldung: **09.01.80 Patentblatt 80/1**

(45) Bekanntmachung des Hinweises auf die Patenterteilung: **15.09.82 Patentblatt 82/37**

(84) Benannte Vertragsstaaten: **AT BE CH DE FR GB IT NL SE**

(56) Entgegenhaltungen:
**DE-A-2 223 375**
**DE-A-2 814 641**

(73) Patentinhaber: **HOECHST AKTIENGESELLSCHAFT, Postfach 80 03 20, D-6230 Frankfurt/Main 80 (DE)**

(72) Erfinder: **Bormann, Dieter, Dr., Johann-Strauss-Strasse 20, D-6233 Kelkheim (Taunus) (DE)**
Erfinder: **Dürckheimer, Walter, Dr., Im Lerchenfeld 45, D-6234 Hattersheim (DE)**
Erfinder: **Schrinner, Elmar, Dr., Hedwigstrasse 2, D-6200 Wiesbaden (DE)**

**Die Akte enthält technische Angaben, die nach dem Eingang der Anmeldung eingereicht wurden und die nicht in dieser Patentschrift enthalten sind.**

EP 0 006 146 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Cephemderivate, Verfahren zu ihrer Herstellung und pharmazeutische Präparate auf Basis dieser Verbindungen

Gegenstand der Erfindung sind Cephemderivate der allgemeinen Formel I

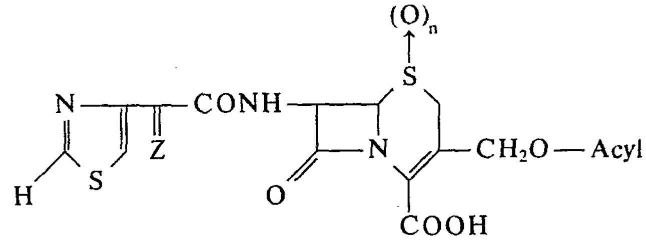

(I)

in der n=0 oder 1 bedeutet, Acyl für aliphatisches Acyl mit 1–5 C-Atomen steht, Z Sauerstoff oder =NOR bedeutet, worin R für Wasserstoff, Alkyl oder Alkenyl mit bis zu 5 C-Atomen steht, sowie deren pharmakologisch verträgliche Salze und Ester.

Gegenstand der Erfindung ist weiterhin ein Verfahren zur Herstellung der Verbindungen der Formel I, das dadurch gekennzeichnet ist, daß man die Cephemverbindungen der allgemeinen Formel II oder deren Ester

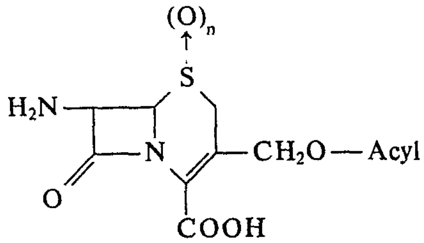

(II)

in der die Reste n und Acyl die oben angegebenen Bedeutungen besitzen, mit Thiazolylcarbonsäuren der allgemeinen Formel III

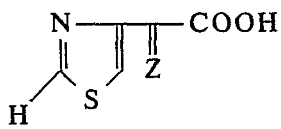

(III)

in der Z die oben angegebene Bedeutung besitzt, worin R außerdem noch eine Schutzgruppe sein kann, in Form ihrer zur Amidbildung befähigten aktivierten Derivate umsetzt oder die Verbindungen der Formel I mit n=0 zu Verbindungen der Formel I mit n=1 oxydiert und gegebenenfalls aus Z in der Bedeutung von =N–OR den Rest R abspaltet, soweit er für eine Schutzgruppe steht.

Aus der DE-A-2 223 375 sind einige Verbindungen bekannt, die sich dadurch von den beanspruchten Verbindungen unterscheiden, daß sie anstelle der Thiazolylgruppe eine Thienyl-, Pyridyl- oder auch eine 1,2-Thiazolylgruppe tragen können. Es war nicht zu erwarten, daß sich die beanspruchten Verbindungen mit einer 1,3-Thiazolylgruppe gerade gegenüber den ihnen sehr nahekommenden 1,2-Thiazolylverbindungen (und auch gegenüber den entsprechenden 1,3-Thiazol-2-yl-verbindungen) durch eine stark überlegene antibakterielle Wirksamkeit auszeichnen würden.

Die Verbindungen der allgemeinen Formel III, ihre Ester und Salze können dadurch erhalten werden, daß man

a) Acetglyoxylester halogeniert und anschließend mit Thioformamid zum Thiazol-4-yl-glyoxylester der allgemeinen Formel III, in der E für einen beliebigen Rest eines Alkohols steht, cyclisiert und diesen zur Thiazol-4-yl-glyoxylsäure sauer oder alkalisch verseift,

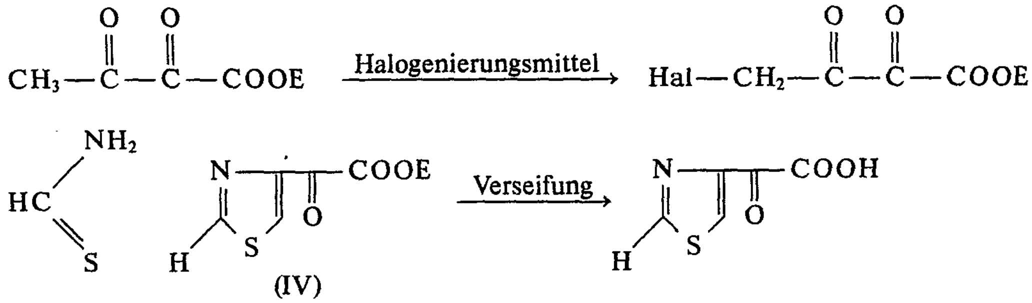

b) Acetessigester halogeniert, das erhaltene Produkt mit Thioformamid zum Thiazol-4-yl-essigsäure-äthylester cyclisiert und diesen zum Thiazol-4-yl-glyoxylester der Formel IV oxydiert

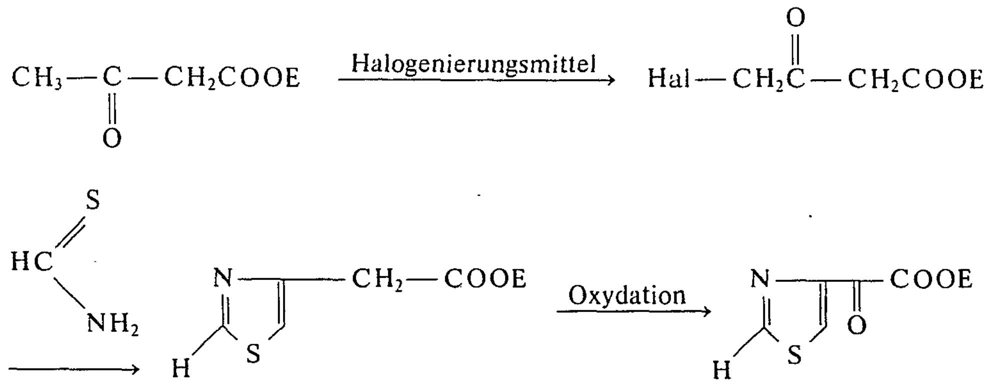


(IV)

c) Thiazol-4-yl-glyoxylsäure oder Ester mit Hydroxylamin zu Verbindungen der Formel II mit Z in der Bedeutung von =NOH umsetzt, und gegebenenfalls den Ester verseift,

d) 2-(Thiazol-4-yl)-2-hydroximinoessigsäure oder die Ester mit Alkylierungsmitteln in Derivate der Formel III überführt, in der Z für =N–OR steht,

e) Thiazol-4-yl-glyoxylsäure oder ihre Ester mit Hydroxylaminderivaten der Formel

$H_2N-OR$

umsetzt und gegebenenfalls die erhaltenen Ester verseift.

f) ω-Halogenacetessigester oxydiert und den erhaltenen Chloracetglyoxylester mit Thioformamid umsetzt

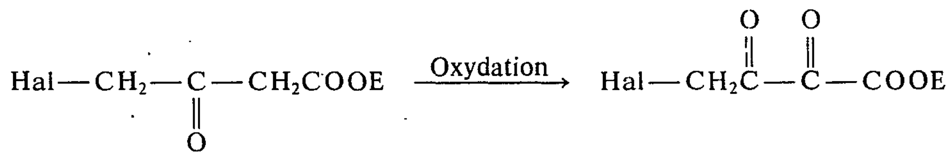


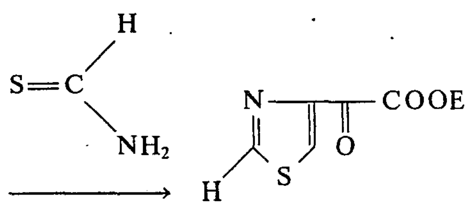

Hal = Chlor oder Brom

g) 2-Aminothiazolcarbonsäuren der Formel

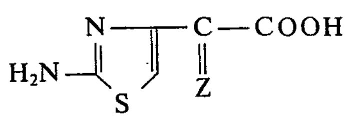

oder deren Ester in situ in an sich bekannter Weise in 2-Diazoniumsalze verwandelt und diese mit einem Reduktionsmittel, vorzugsweise unterphosphoriger Säure in Gegenwart von Cu-Pulver reduziert,

h) 2-Aminothiazolcarbonsäuren der Formel

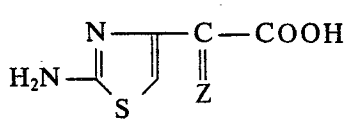

oder deren Ester in einer Sandmeyer-Reaktion in die 2-Bromthiazolderivate überführt und diese mit katalytisch erregtem Wasserstoff enthalogeniert.

Als pharmakologisch verträgliche Salze der Cephemverbindungen der Formel I kommen anorganische und organische Salze in Betracht, insbesondere die Alkali- und Erdalkalisalze, vorzugsweise die Natrium-, Magnesium- und Calciumsalze, die Triäthylammoniumsalze oder das Procainsalz.

Als pharmakologisch anwendbare Ester seien besonders die leicht spaltbaren Ester, wie beispielsweise die Acetoxymethylester, Pivaloyloxymethylester sowie der Phthalidester genannt.

Steht Z in der allgemeinen Formel I für =NOR, so kommen als Reste R Wasserstoff oder Alkyl- oder Alkenylreste mit 1 bis 5 C-Atomen, vorzugsweise 1–3 C-Atomen in Betracht, wie z. B. Methyl, Äthyl, Allyl, Vinyl, Propyl, Isopropyl, Butyl, Isobutyl, Pentenyl, vorzugsweise Methyl, Äthyl und Allyl. Besonders bevorzugt sind für R die Bedeutungen von Wasserstoff und Methyl.

Als Acylreste mit 1 bis 5 C-Atomen kommen beispielsweise die Formyl-, Acetyl- oder Propionylgruppe, insbesondere aber die Acetylgruppe, in Betracht.

Die für die erfindungsgemäß durchzuführenden Reaktionen einzusetzenden Cephemverbindungen der Formel

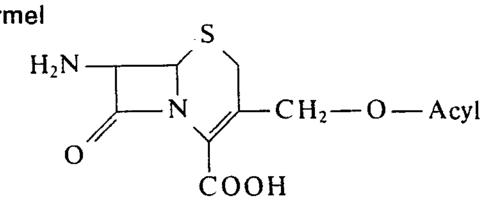

oder ihrer Ester sind literaturbekannt.

Die Ausgangsverbindungen der Formel

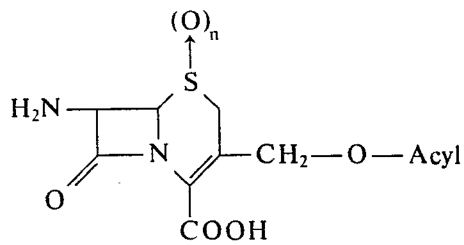

können durch Oxydation der entsprechenden 7-Aminocephalosporansäurederivate in der weiter unten beschriebenen Weise erhalten werden.

Die zur Acylierung verwendeten Carbonsäuren der Formel III sind neue Verbindungen, die sich in guten Ausbeuten herstellen lassen. Ausgangspunkt für die Synthese nach a) ist der Acetglyoxylester, der entsprechend den Angaben aus der Literatur aus Acetessigester zugänglich ist.

Zur Bildung des Thiazolrings sind in der Literatur mehrere Wege beschrieben worden, beispielsweise die Umsetzung von Chlorketoderivaten mit Thioformamid entsprechend der folgenden Gleichung.

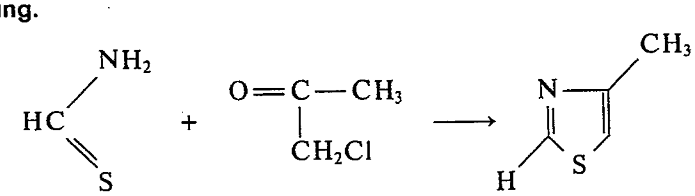

Acetylglyoxylsäureester lassen sich glatt und in hohen Ausbeuten in Halogenacetglyoxylester umwandeln. Als Halogenierungsmittel haben sich besonders Sulfurylchlorid oder elementares Brom bewährt.

Die Halogenierung erfolgt in einem Lösungsmittel. Als Lösungsmittel haben sich besonders die halogenierten Kohlenwasserstoffe bewährt wie beispielsweise Methylenchlorid, Chloroform oder Äthylendichlorid.

Die Umsetzung kann in einen weiten Temperaturbereich durchgeführt werden. Zur Erzielung hoher Ausbeuten an Monohalogenacetglyoxylester ist ein Temperaturbereich von –20° bis +20° bevorzugt.

Hat man zur Halogenierung elementares Brom verwendet, so kann der Bromacetglyoxylester als Rohmaterial direkt weiter mit Thioformamid umgesetzt werden.

Alternativ erhält man den Brom- oder Chloracetglyoxylester durch Oxydation von ω-Brom- oder ω-Chloracetessigester (Verfahren f). Als Oxydationsmittel hat sich besonders Selendioxyd bewährt, das in einem mit Wasser azeotrop destillierenden halogenierten Kohlenwasserstoff wie beispielsweise Chloroform oder Äthylendichlorid angewendet wird, wobei das gewünschte Produkt nach Abtrennung von rohem Selen in hohen Ausbeuten anfällt und direkt oder, falls gewünscht, nach Destillation mit Thioformamid umgesetzt werden kann.

Zur Erzielung guter Ausbeuten empfiehlt es sich, das Thioformamid in mindestens äquimolarer Menge einzusetzen, wobei darauf zu achten ist, daß das Thioformamid nach literaturbekannten Verfahren frisch bereitet wird.

Die Umsetzung läßt sich auf verschiedene Weise durchführen. Eine bevorzugte Methode besteht darin, das frisch bereitete Thioformamid in Lösung vorzulegen und die Halogenverbindung zur

Reaktionsmischung zulaufen zu lassen.

Als Lösungsmittel für diese Umsetzung haben sich verschiedene organische Lösungsmittel wie beispielsweise Alkohole, aber auch Mischungen von organischen Lösungsmitteln mit Wasser bewährt. Geeignet sind Mischungen aus Alkohol und Wasser, insbesondere Äthanol und Wasser. Bevorzugt wird die Reaktion in Äthanol durchgeführt.

Die Reaktion kann in einem weiten Temperaturbereich ausgeführt werden, beispielsweise bei −50° bis +80°. Besonders ist der Temperaturbereich von −20° bis +30° geeignet. Die Isolierung der Endprodukte erfolgt in an und für sich bekannter Weise z. B. durch Extraktion des Reaktionsprodukts und anschließende Destillation.

Die destillativ gereinigten 1,3-Thiazol-4-yl-glyoxylsäureester werden in an und für sich bekannter Weise zu der 1,3-Thiazol-4-yl-glyoxylsäure verseift, beispielsweise durch Umsetzen mit wäßrigen Alkali- oder Erdalkalihydroxyden, wie beispielsweise Natronlauge. Darüber hinaus stellen die Ester, wie aber auch die Säuren, wertvolle Ausgangsmaterialien für die Herstellung von Verbindungen der Formel III dar, in denen Z für die Gruppierung =NOR steht, wie es in den Verfahrensvarianten c, d und e erläutert ist.

Der bei der Anwendung des Verfahrens b eingesetzte 1,3-Thiazol-4-yl-essigsäure-äthylester ist literaturbekannt. Die Oxydation der Essigsäuregruppierung zu der entsprechenden Glyoxylsäuregruppierung gelingt glatt und in guten Ausbeuten, wenn man die Oxydation in einem inerten organischen Lösungsmittel durchführt, das in der Lage ist, das bei der Oxydation entstandene Wasser azeotrop zu entfernen. Als solche Lösungsmittel eignen sich Benzol und die halogenierten Kohlenwasserstoffe. Besonders bewährt hat sich das Dichloräthylen.

Die Oxydation wird durch Zugabe eines Oxydationsmittels durchgeführt. Als besonders einfach hat sich die Oxydation mit Selendioxyd herausgestellt, wobei man die Reaktionstemperatur durch den Siedepunkt des sich bildenden Azeotrops einstellen kann.

Den Fortgang der Oxydation kann man an der Wasserabscheidung bequem verfolgen. Die Aufarbeitung kann beispielsweise durch Filtration und fraktionierte Destillation erfolgen.

Die auf einem der Wege a, b oder f hergestellten 1,3-Thiazol-4-ylglyoxylester werden durch einfache Verseifung in die 1,3-Thiazol-4-yl-glyoxylsäuren überführt. Diese können als solche nach entsprechender Aktivierung mit den Cephemverbindungen der Formel I umgesetzt werden. Zur Aktivierung eignen sich eine Vielzahl von Reagenzien; besonders einfach erfolgt die Bildung eines symmetrischen oder unsymmetrischen Anhydrids in einem die Reaktion nicht behindernden Lösungsmittel, insbesondere in halogenierten Kohlenwasserstoffen, wie beispielsweise Methylenchlorid oder Chloroform, in einem weiten Temperaturbereich.

Ein bevorzugtes Verfahren zur Aktivierung besteht darin, die 1,3-Thiazol-4-ylglyoxylsäure mit Chlorameisensäureester oder Pivaloylsäurechlorid umzusetzen, wobei die Säure in ein Salz überführt werden muß. Es hat sich gezeigt, daß die Reaktion glatt verläuft, wenn man die Säure in halogenierten Kohlenwasserstoffen, wie beispielsweise Methylenchlorid, suspendiert und mit organischen Basen, wie beispielsweise Triäthylamin, in das Triäthylammoniumsalz überführt. Alternativ läßt sich das Alkalisalz der 1,3-Thiazol-4-ylglyoxylsäure einsetzen, wobei sich die Zugabe von katalytischen Mengen einer tertiären Base, wie z. B. N,N-Dimethylanilin, als günstig erwiesen hat.

Man kann auch mit Kondensationsmitteln, wie beispielsweise Dicyclohexylcarbodiimid, aus der 1,3-Thiazol-4-ylglyoxylsäure das innere Anhydrid bilden, das anschließend mit den Aminocephemcarbonsäurederivaten der Formel II umgesetzt wird.

Die Acylierung der Cephemverbindungen der Formel II mit den 1,3-Thiazol-4-ylglyoxylsäurederivaten kann unter verschiedenen experimentellen Bedingungen durchgeführt werden. So lassen sich die Aminocephemderivate der Formel II in den verschiedensten Lösungsmitteln acylieren. Als Lösungsmittel eignen sich beispielsweise organische Lösungsmittel, wie halogenierte Kohlenwasserstoffe, wie z. B. Methylenchlorid, Chloroform oder auch tertiäre Amide, wie Dimethylformamid oder Dimethylacetamid.

Zur guten Reaktionsführung ist es wünschenswert, die Aminolactamderivate der Formel II in Lösung zu bringen.

Im Fall der Aminocephemester der allgemeinen Formel II erfolgt die Umsetzung in organischen Lösungsmitteln, in denen die Ester zumeist gut löslich sind. Als Beispiele für solche Lösungsmittel seien die halogenierten Kohlenwasserstoffe oder die tertiären Amide genannt.

Als Ester im Sinne der Erfindung kommen z. B. solche Verbindungen der Formel II in Betracht, in denen die Estergruppe für niedrigmolekulares Alkyl, vorzugsweise tertiäres Butyl oder substituiertes Methyl steht, wobei die Methylgruppe insbesondere durch Trichlormethyl, Acyloxy, vorzugsweise Acetoxy oder Pivaloyloxy, durch einen oder zwei Phenylreste, die ihrerseits beispielsweise durch niedrigmolekulares Alkoxy, vorzugsweise Methoxy, oder die Nitrogruppe substituiert sein können, oder den Phthalidester steht. Als Beispiele für die vorstehenden Definitionen seien insbesondere genannt die tert.-Butylester, die Trichloräthyl-, p-Methoxybenzyl-, die Benzhydryl-, Acetoxymethyl-, Pivaloxyloxymethylester oder die Phthalidester.

Im Falle einer Verwendung der Aminocephemcarbonsäuren der allgemeinen Formel II müssen die Verbindungen in Lösung gebracht werden. Als geeignete Methoden haben sich der Zusatz von Basen, aber auch die Silylierung bewährt. Als Silylierungsmittel eignen sich die gängigen Reagenzien,

insbesondere das Trimethylchlorsilan, das in Gegenwart der stöchiometrischen Menge Base angewendet wird, ganz besonders aber das O,N-Bistrimethylsilylacetamid, das ohne Basenzusatz verwendet werden kann. Zur Erzielung guter Ausbeuten ist es ratsam, das Silylierungsmittel im Verhältnis von etwa 2 Äquivalenten Silyl pro Mol Aminocephemverbindung der Formel II anzuwenden. Als für die Auflösung der 7-ACS sowie einer Vielzahl von 7-Amino-$\Delta^3$-cephem-4-carbonsäuren geeignete Basen kommen vor allem organische Basen in Betracht. So haben sich für die Herstellung von Lösungen in organischen Lösungsmitteln insbesondere die tertiären Amine, wie Triäthylamin, N,N-Dimethylanilin oder N-Methylmorpholin bewährt.

Die Basen werden im allgemeinen in mindestens stöchiometrischer Menge, bezogen auf die gewünschte Umsetzung, zugesetzt. Es empfiehlt sich jedoch, einen Überschuß an Base von beispielsweise etwa 20 bis 80% zu verwenden.

Insbesondere bei gegen Basen empfindlichen Verbindungen der Formel II läßt sich durch kontinuierliche Zugabe der Base je nach Reaktionsverlauf der pH von etwa 4 bis 8, vorzugsweise 6 bis 7, konstant halten.

Die Auflösung der Aminolactamderivate der Formel II kann in einem weiten Temperaturbereich erfolgen. Bei gegen Basen empfindlichen Derivaten empfiehlt es sich jedoch, einen Temperaturbereich von etwa 0 bis 15° zu wählen.

Zu den in Lösung oder gegebenenfalls in Suspension vorliegenden Amino-cephemderivaten der Formel II wird die aktivierte 1,3-Thiazol-4-yl Glyoxylsäure gegeben. Die Umsetzung erfolgt in an und für sich bekannter Weise bei Temperaturen, wie sie für die Herstellung von Carbonsäureamiden aus reaktiven Carbonsäurederivaten der Formel III gebräuchlich sind.

Als geeignet hat sich ein Temperaturintervall von −50 bis +30° erwiesen, bevorzugt wird die Umsetzung bei −20 bis 0° durchgeführt.

Das aktivierte Säurederivat der Formel III wird zur Erzielung hoher Ausbeuten in mindestens stöchiometrischer Menge eingesetzt. Ein Überschuß von etwa 5−25% kann sich als zweckmäßig erweisen.

Die Isolierung der Acylierungsprodukte kann nach an sich bekannten Methoden erfolgen. So lassen sich beispielsweise die erhaltenen Säurederivate der Formel I, gegebenenfalls nach Abdampfen des organischen Lösungsmittels, in Wasser aufnehmen und durch Zugabe von Mineralsäuren ausfällen. Als Mineralsäuren eignen sich besonders verdünnte Säuren, wie verdünnte Salzsäure oder Schwefelsäure. Die Amidocephemsäuren der Formel I fallen in den meisten Fällen als amorphe Feststoffe oder in kristalliner Form aus. Sie lassen sich auch durch Extraktion bei pH 2 bis 1 als freie Säuren abtrennen. Als Extraktionsmittel können verschiedene, mit Wasser nicht mischbare organische Lösungsmittel verwendet werden, beispielsweise halogenierte Kohlenwasserstoffe, wie Methylenchlorid, oder Ester, wie beispielsweise Essigsäureäthylester oder Essigsäure-n-butylester, aber auch Ketone, wie Methylisobutylketon.

Aus den Extrakten werden die entstandenen Amidocephemsäuren der Formel I gewonnen, beispielsweise durch Abdampfen des Lösungsmittels und Anreiben; beispielsweise mit Äther.

Die unter g) beschriebene Umsetzung kann beispielsweise so durchgeführt werden, daß man die entsprechenden 2-Aminothiazolcarbonsäuren oder deren Ester in saurer Lösung unter Kühlung und Zusatz von Natriumnitrit-Lösung diazotiert und das intermediär gebildete Diazoniumsalz in der für die Reduktion von Diazoniumsalzen bekannten Weise reduziert, beispielsweise durch Verwendung von unterphosphoriger Säure. Die Isolierung kann z. B. im Falle der Ester durch Extraktion der neutralisierten Lösung erfolgen. Die freien Säuren kann man daraus beispielsweise durch alkalische Verseifung erhalten.

Wenn man nach h) arbeitet, kann die Diazoniumverbindung auch durch Behandlung mit Bromwasserstoffsäure und Cu-Pulver in die entsprechende Bromverbindung überführt werden, die durch katalytisch erregten Wasserstoff, z. B. durch Reduktion in Gegenwart von Raney-Nickel und einer Base quantitativ enthalogeniert wird.

Die auf einem der Wege a, b oder f hergestellten 1,3-Thiazol-4-ylglyoxylsäureester oder die dazugehörigen Carbonsäurederivate eignen sich entsprechend den Verfahren c, d und e zur Herstellung der Verbindungen der Formel III, in denen Z für eine N−OR-Gruppe steht.

Zur Durchführung dieser Reaktion setzt man die 1,3-Thiazol-4-yl-glyoxylsäureester oder die entsprechenden Carbonsäuren mit Hydroxylamin (Verfahren c) oder den Hydroxylamin-O-alkyl- bzw. Hydroxylamin-O-arylderivaten um (Verfahren e). Die Hydroxylaminderivate der allgemeinen Formel

$$H_2NOR$$

in der R die eingangs angegebenen Bedeutungen besitzt, sind literaturbekannt oder lassen sich auf den in der Literatur angegebenen Wegen bequem herstellen. Die Umsetzung des Hydroxylamins bzw. der Hydroxylaminderivate der Formel

$$H_2NOR$$

mit der 1,3-Thiazol-4-yl-glyoxylsäure bzw. dem Ester erfolgt in der literaturbekannten Weise, wie es

beispielsweise im »Organicum«, VEB Deutscher Verlag der Wissenschaften, Auflage 1967, S. 369 ff., für die Reaktion von Carbonylgruppen mit Carbonylreagenzien angegeben ist. Die durch diese Reaktion erhaltenen 2-(1,3-Thiazol-4-yl)-2-oximino-essigsäuren enthalten die Oximgruppierung in syn-Position, bezogen auf die COOH-Gruppe, wie es durch die Formel VI

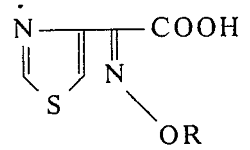

(VI)

dargestellt ist.

Steht in der obigen Formel VI R für Wasserstoff, so läßt sich die so erhaltene Verbindung in Gegenwart von organischen oder anorganischen Basen leicht und in hohen Ausbeuten alkylieren (Verfahrensvariante d). Als Alkylierungsmittel kommen die Dialkylsulfate, wie z. B. das Dimethylsulfat, aber auch die Alkylhalogenide, wie beispielsweise Methyljodid, Benzylbromid, p-Methoxybenzylchlorid oder das Triphenylchlormethan in Betracht. Die für die Alkylierung als Hilfsstoffe verwendeten Basen können je nach dem Reaktionstyp gewählt werden aus den Alkali- oder Erdalkalihydroxyden, wie z. B. Natrium- oder Kaliumhydroxyd, oder den organischen Basen, wie z. B. Triäthylamin. Die Alkylierung erfolgt in einem Lösungsmittel oder Lösungsmittelgemisch. Besonders vorteilhaft verwendet man Wasser oder Mischungen aus Alkohol und Wasser oder die halogenierten Kohlenwasserstoffe, wie beispielsweise Methylenchlorid oder Chloroform.

Die Aufarbeitung der Endprodukte erfolgt nach üblichen Laboratoriumserfahrungen, wobei bei Verwendung der Ester diese nicht gereinigt werden müssen, sondern in die freien Säuren überführt werden können.

Die Umsetzung der 1,3-Thiazol-4-yl-essigsäurederivate der Formel III, in der Z für NOR steht, wobei R nicht Wasserstoff bedeuten kann, mit den Cephemverbindungen der Formel II erfolgt in an und für sich bekannter Weise, wie sie bereits vorstehend beschrieben wurde, beispielsweise nach Aktivierung der Carbonsäuregruppe durch Überführung in eine zur Amidbildung befähigte Gruppierung, wie vorzugsweise die Überführung in einen Aktivester, beispielsweise den Hydroxybenztriazolester, oder durch Überführung in ein Säurehalogenid, wie vorzugsweise ein Säurechlorid, oder durch Überführung in ein symmetrisches oder unsymmetrisches Anhydrid.

Bei den Aktivierungsreaktionen muß durch die Wahl möglichst milder Reaktionsbedingungen verhindert werden, daß sich die syn-Oxime in die trans-Oximderivate umlagern, die durch die folgende Formel wiedergegeben werden.

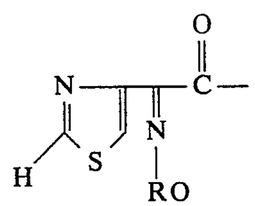

Die Durchführung der Aktivierung erfolgt daher bei möglichst niedrigen Temperaturen im Bereich von $-50°$ bis $+30°$, besonders bevorzugt erfolgt die Aktivierung bei $-10°$ bis $+10°$.

Erfindungsgemäß ist somit die Herstellung von Verbindungen, in denen die Oximgruppierung =NOR syn-Konfiguration aufweist, ganz besonders bevorzugt.

Hat man das symmetrische Anhydrid für die Reaktion verwendet, so wird die bei der Acylierung frei gewordene Seitenkettensäure beispielsweise durch Extraktion oder Fällung abgetrennt.

Zur Herstellung der Verbindungen der Formel I, in denen Z die Bedeutung =N−OH besitzt, ist es ratsam, die NOH-Gruppe in der Carbonsäure der Formel III durch eine für derartige Umsetzungen übliche Schutzgruppe, wie beispielsweise eine Tetrahydropyranylgruppe oder eine Triarylmethylgruppe, vorübergehend zu blockieren. Besonders bewährt hat sich als derartige Schutzgruppe beispielsweise die Triphenylmethylgruppe, die nach Isolierung der Endprodukte durch literaturbekannte Verfahren, wie beispielsweise durch Spaltung mit wäßriger Ameisensäure, wieder abgespalten werden kann. Bei der vorübergehenden Blockierung der N−OH-Gruppe in den Thiazol-4-yl-säuren der Formel III empfiehlt es sich, die Ester der Formel III einzusetzen und diese anschließend durch alkalische Hydrolyse in die an der N−OH-Gruppe geschützten Carbonsäuren zu verseifen.

Eine alternative Darstellungsweise für die Verbindungen der Formel I, in denen n=1 ist, d. h. die Herstellung der Cephem-S-oxyde, besteht darin, die Verbindungen der Formel I mit n=0 zu oxydieren.

Die Oxydation von Cephemderivaten am Schwefel ist mehrfach beschrieben, wobei $\alpha$- und $\beta$-Oxyde entstehen können (E. Flynn, Cephalosporins and Penicillins, Chemistry and Biology, Academic Press, 1972, S. 135 ff.), je nachdem, welches Oxydationsmittel benutzt wird.

Benutzt man beispielsweise Peressigsäure in Eisessig, so erhält man die β-S-oxyde. Die Reaktionstemperatur der Reaktion ist nicht kritisch, jedoch empfiehlt es sich, zur Vermeidung von unerwünschten Folgereaktionen, die Oxydation bei Raumtemperatur durchzuführen und das Oxydationsmittel in mindestens stöchiometrischer Menge zuzusetzen, wobei ein Überschuß von 10—100% häufig zweckmäßig ist, solange die Temperatur der Reaktion nicht wesentlich gesteigert wird.

Die Isolierung der auf diesem Weg hergestellten Cephem-S-oxyde der Formel I mit n = 1 bereitet keine Schwierigkeiten und kann beispielsweise durch Fällung und anschließende Filtration, oder auch durch Extraktion erfolgen.

Die Amidocephemverbindungen der Formel I lassen sich auch durch nachträgliche Veresterung nach literaturbekannten Verfahren in die physiologisch verträglichen Ester der Formel I überführen. So erhält man beispielsweise die Acetoxymethyl- oder Pivaloyloxymethylester durch Umsetzung der Alkalisalze, vorzugsweise der Natriumsalze, oder der Ammoniumsalze, vorzugsweise der Triäthylammoniumsalze, mit den entsprechenden Halogenmethylacylverbindungen, wie beispielsweise Chlormethylacetat, Chlormethylpropionat oder Pivaloylsäurechlormethylester.

Soweit die Ester, insbesondere die physiologisch verträglichen Ester, bereits bei der Acylierung angefallen sind, erübrigt sich eine nachträgliche Veresterung der Carboxylgruppe.

Die bei der erfindungsgemäßen Umsetzung direkt angefallenen Ester, wie z. B. p-Methoxybenzyl-, p-Nitrobenzyl-, tert.-Butyl- oder Benzhydrylester können auch in literaturbekannter Weise in die freien Carbonsäuren der Formel I überführt werden.

Die Cephemderivate der Formel I sind wertvolle Antibiotica, die sich überraschend gut zur Bekämpfung grampositiver und vor allem gramnegativer Infekte eignen und darüber hinaus auch gegen penicillinasebildende Staphylokokken unerwartet gut wirksam sind.

Die erfindungsgemäßen Verbindungen können als solche oder zusammen mit den therapeutisch üblicherweise eingesetzten Hilfs- und Zusatzstoffen, wie z. B. Traganth, Milchzucker, Talkum, Lösungsmitteln, etc. in Form galenischer Zubereitungen, wie z. B. Tabletten, Dragees, Kapseln, Suspensionen, Lösungen etc. peroral, vorzugsweise jedoch parenteral eingesetzt werden, wobei der Wirkstoff in der Regel in einer Menge von etwa 50 bis 1000 mg, vorzugsweise etwa 100 bis 500 mg, in einer Verabreichungseinheit enthalten ist.

Für die parenterale Anwendung kommen die für den therapeutischen Einsatz bekannten Lösungsmittel, insbesondere eine Lösung in Wasser in Betracht.

Es ist auch möglich, die erfindungsgemäßen Verbindungen mit anderen Wirkstoffen zu kombinieren. So können beispielsweise andere Antibiotica appliziert werden, wie z. B. solche aus der Reihe der Penicilline, Cephalosporine, oder Verbindungen, die die Symtomatik bakterieller Infektionen beeinflussen, wie z. B. Antipyretika, Antiphlogistica oder Analgetica.

Neben den in den Beispielen beschriebenen Cephemderivaten der Formel I lassen sich beispielsweise auch die folgenden Verbindungen nach den erfindungsgemäßen Verfahren herstellen:

7-(2-(1,3-Thiazol-4-yl)-2-syn-äthoximinoacetamido)-cephalosporansäure
7-(2-(1,3-Thiazol-4-yl)-2-syn-propoximinoacetamido)-cephalosporansäure
7-(2-(1,3-Thiazol-4-yl)-2-syn-butoximino-acetamido)-cephalosporansäure
7-(2-(1,3-Thiazol-4-yl)-2-syn-pentyloximinoacetamido)-cephalosporansäure
7-(2-(1,3-Thiazol-4-yl)-2-syn-allyloximinoacetamido)-cephalosporansäure
7-(2-(1,3-Thiazol-4-yl)-2-syn-allyloximinoacetamido)-cephalosporansäure-β-S-oxyd
7-(2-(1,3-Thiazol-4-yl)-2-syn-äthoximinoacetamido)-cephalosporansäure-β-S-oxyd
7-(2-(1,3-Thiazol-4-yl)-2-syn-pentyloximino-acetamido)-cephalosporansäure-β-S-oxyd
7-(2-(1,3-Thiazol-4-yl)-2-syn-hydroximinoacetamido)-cephalosporansäure-β-S-oxyd
7-(2-(1,3-Thiazol-4-yl)-2-syn-i-propyloximinoacetamido)-cephalosporansäure
7-(2-(1,3-Thiazol-4-yl)-2-syn-methoximinoacetamido)-cephalosporansäure-α-S-oxyd.

Auch diese Verbindungen können als solche, oder beispielsweise nach Überführung in Salze, insbesondere ihre Natrium-, Calzium- oder Magnesiumsalze oder auch als Ester, insbesondere ihre Acetoxymethyl- oder Pivaloyloxymethylester, eingesetzt werden.

Die folgenden Ausführungsbeispiele dienen zur weiteren Erläuterung der Erfindung, schränken sie jedoch nicht darauf ein.

## Beispiel 1

### 7-(2-(1,3-Thiazol-4-yl)-2-syn-methoximinoacetamido)-cephalosporansäure

Zu einer Suspension von 2,6 g 2-(1,3-Thiazol-4-yl)-2-syn-methoximino-essigsäure in 50 ml Methylenchlorid wird unter Feuchtigkeitsausschluß eine Lösung von 1,58 g Dicyclohexylcarbodiimid in 10 ml Methylenchlorid bei 0° zugetropft. Die Reaktionsmischung wird nach 2 Stunden bei 0° auf —5° gekühlt, mit einer Lösung von 1,9 g 7-Aminocephalosporansäure in 25 ml Methylenchlorid und 1,5 g

Triäthylamin versetzt, 3 Stunden ohne Kühlung gerührt und filtriert, wobei der entstandene Harnstoff entfernt wird.

Das Filtrat wird zur Trockne eingeengt, der Rückstand in wenig Wasser aufgenommen, auf pH 4 gestellt und die nicht umgesetzte 7-ACS abgetrennt.

Die Mutterlauge wird auf pH 1 mit 2n HCl angesäuert, gut gekühlt und der ausgefallene Kristallbrei isoliert. Nach dem Trocknen wurde das Gemisch aus der 2-(1,3-Thiazol-4-yl)-2-syn-methoximinoessigsäure und der gewünschten 7-(2-(1,3-Thiazol-4-yl)-2-syn-methoximino-acetamido)-cephalosporansäure mit wenig Methanol versetzt, gut gekühlt, wobei die Cephemverbindung in nahezu farblosen Kristallen auskristallisiert. Die Kristalle werden isoliert, mit wenig kaltem Methanol nachgewaschen und man erhält die 7-(2-(1,3-Thiazol-4-yl)-2-syn-methoximinoacetamido)-cephalosporansäure als nahezu farblose Kristalle vom Schmp. 140—142° (Zers.).

DC (BuOH zu $H_2O$ zu Äthanol zu Eisessig = 5 : 2 : 1,5 : 1,5): Rf = 0,43.

IR in KBr: Lactam — CO 1750 $cm^{-1}$.

NMR: Thiazolprotonen bei $\delta$ = 7,93 und 9,13 ppm (gemessen in $((CD_3)_2SO)$, jeweils Dubletts J = 2 Hz.

## Beispiel 2

### 7-(2-(1,3-Thiazol-4-yl)-2-syn-methoximinoacetamido)-cephalosporansäure

Zu einer Suspension von 7,4 g 2-(1,3-Thiazol-4-yl)-2-syn-methoximino-essigsäure in 75 ml Methylenchlorid werden 3,6 ml Dimethylacetamid unter Feuchtigkeitsausschluß bei —10° hinzugefügt und anschließend bei —10° 25 ml einer 21,3prozentigen Phosgen-Lösung in Toluol innerhalb von 15 Min. zugetropft. Die Reaktionsmischung rührt man 2 Stunden, wobei sich das 2-(1,3-Thiazol-4-yl)-2-syn-methoximinoessigsäurechlorid bildet. Zu der Reaktionsmischung wird anschließend eine Lösung von 10,85 g 7-Aminocephalosporansäure in 200 ml Methylenchlorid, 10,3 ml Triäthylamin und 6,8 g Pyrrolidon bei —10° zugetropft, 2 Stunden bei —5° gerührt, anschließend die organische Phase mit Wasser versetzt, auf pH 7 gestellt und die wäßrige Phase abgetrennt. Die wäßrige Phase wird auf pH 3,5 gestellt, filtriert und anschließend auf pH 1 angesäuert. Die ausgefallenen Kristalle werden durch Extraktion mit Essigester isoliert, die Essigesterphase getrocknet, eingeengt und der Rückstand in wenig Methanol gelöst. Beim Abkühlen kristallisiert die 7-(2-(1,3-Thiazol-4-yl)-2-syn-methoximinoacetamido)-cephalosporansäure in farblosen Kristallen vom Schmp. 140—142° aus.

## Beispiel 3

### 7-(2-(1,3-Thiazol-4-yl)-2-syn-hydroximinoacetamido)-cephalosporansäure

a) 2,9 g 2-(1,3-Thiazol-4-yl)-2-syn-triphenylmethoximinoessigsäure werden im Hochvakuum extrem getrocknet, anschließend mit 40 ml Toluol und 0,54 ml Dimethylformamid versetzt, die Reaktionsmischung auf —10° gekühlt und mit 5 ml einer 32prozentigen Phosgenlösung in Toluol versetzt. Nach 2,5 Stunden bei —10° wird eine Lösung von 1,9 g 7-Amino-cephalosporansäure in 30 ml Methylenchlorid und 4 ml Triäthylamin zugetropft, weitere 2 Stunden gerührt, zuletzt ohne Kältebad, so daß die Temperatur auf +20°C stieg, und anschließend mit 60 ml Eiswasser versetzt. Die organische Phase wird abgetrennt, die wäßrige Phase nochmals mit Methylenchlorid gewaschen. Die vereinten organischen Phasen werden über $Na_2SO_4$ getrocknet, filtriert, das Lösungsmittel abgezogen und der Rückstand mit Äther verrieben. Dabei bildet sich ein Feststoff, der isoliert, mit Äther nachgewaschen und getrocknet wird. Man erhält die 7-(2-(1,3-Thiazol-4-yl)-2-syn-triphenylmethoximinoacetamido)-cephalosporansäure vom Schmp. 135—140° Zers.

DC (Fließmittel wie Beispiel 1): Ref 0,71.

IR in KBr: Lactam CO: 1780 $cm^{-1}$.

b) 2,03 g der 7-(2-(1,3-Thiazol-4-yl)-2-syn-triphenylmethoximinoacetamido)-cephalosporansäure werden in eine Mischung aus 20 ml 100prozentige Ameisensäure und 5 ml Wasser eingetragen, 2 Stunden bei Raumtemperatur gerührt und anschließend das ausgefallene Triphenylcarbinol durch Filtration entfernt. Das Filtrat wird zur Trockne eingeengt, der Rückstand mit 20 ml Äther und 20 ml Isopropanol versetzt und verrieben. Es bildet sich ein Feststoff, der isoliert, mit Äther nachgewaschen und getrocknet wird. Man erhält die 7-(2-(1,3-thiazol-4-yl)-2-syn-hydroximinoacetamidocephalosporansäure als cremefarbenen Feststoff, der bis 250° nicht schmilzt.

DC (Fließmittel Essigester zu Äthanol zu $H_2O$ zu HCOOH 60 : 25 : 15 : 1): Rf 0,57.

IR in KBr: Lactam CO 1770 $cm^{-1}$.

NMR $((CD_3)_2SO)$: Thiazolprotonen $\delta$ = 7,81 und 9,08 ppm, jeweils Dubletts J = 1,5 Hz.

Beispiel 4

7-(2-(1,3-Thiazol-4-yl)-glyoxylamido)-cephalosporansäure

Eine Lösung von 3,14 g 1,3-Thiazol-4-yl-glyoxylsäure in 30 ml Methylenchlorid und 2,2 g Triäthylamin werden bei 0° unter Feuchtigkeitsausschluß mit einer Lösung von 2,5 g Pivaloylsäurechlorid in 10 ml Methylenchlorid versetzt und 1 Stunde gerührt. Anschließend wird zu der Reaktionsmischung eine Lösung von 5,44 g 7-Aminocephalosporansäure in 30 ml Methylenchlorid und 6,06 g Triäthylamin zugetropft, 30 Min. bei 0° und anschließend 2 Stunden bei Raumtemperatur gerührt und danach die Lösungsmittel entfernt.

Der Rückstand wird in 100 ml Wasser aufgenommen, zur Abscheidung von eventuell vorhandener 7-Aminocephalosporansäure zunächst auf pH 3,5 gestellt, filtriert, anschließend auf pH 1 gestellt und die ausgefallenen Kristalle isoliert. Man erhält so die 7-(2-(1,3-Thiazol-4-yl)-glyoxylamido)-cephalosporansäure als beigefarbenen Feststoff.

DC (Butanol, Wasser, Äthanol, Eisessig wie 5 : 2 : 1,5 : 1,5): Rf 0,38.

Lactam CO: 1772 $cm^{-1}$.

Eine weitere Menge kann man durch Extraktion mit Essigester und anschließendes Verreiben mit Äther als cremefarbenen Feststoff erhalten.

NMR $(CD_3)_2SO$): Thiazolprotonen bei 8,85 und 9,23 ppm, jeweils Dubletts J = 2 Hz.

Beispiel 5

7-(2-(1,3-Thiazol-4-yl)-2-syn-methoximinoacetamido)-cephalosporansäure-β-S-oxyd

Eine Suspension von 1 g der im Beispiel 1 hergestellten 7-(2-(1,3-Thiazol-4-yl)-2-syn-methoximinoacetamido)-cephalosporansäure in 10 ml 80% Essigsäure wird bei Raumtemperatur mit einer Lösung von 800 mg 40% Peressigsäure in Eisessig versetzt. Die Reaktionsmischung wird 30 Min. bei Raumtemperatur nachgerührt, anschließend werden die ausgefallenen Kristalle isoliert, mit Methanol und Äther gewaschen. Man isoliert das 7-(2-(1,3-Thiazol-4-yl)-2-syn-methoximinoacetamido)-cephalosporansäure-β-S-oxyd als farblose Kristalle vom Schmp. 175—176° (Zers.).

DC im Fließmittel BuOH, $H_2O$, $C_2H_5OH$, $CH_3COOH$ = 5 : 2 : 1,5 : 1,5: Rf 0,29.

IR in KBr: Lactam — CO bei 1785 $cm^{-1}$.

NMR: Thiazolprotonen bei δ 7,96 und 9,13 ppm ($(CD_3)_2SO$), jeweils Dubletts J = 2 Hz.

Beispiel 6

7-(2-(1,3-Thiazol-4-yl)-glyoxylamido)-cephalosporansäure-β-S-oxyd

Eine Lösung von 1,5 g der im Beispiel 5 hergestellten 7-(1,3-Thiazol-4-yl-glyoxylamido)-cephalosporansäure in 25 ml 80% Essigsäure wird unter leichtem Kühlen mit 1 g 40% Peressigsäure in Eisessig versetzt, so daß die Temperatur nicht über 25° steigt.

Die Reaktionsmischung wird nach 30 Min. mit 10 ml Methanol versetzt, und anschließend im Vakuum zur Trockne eingeengt. Der Rückstand liefert beim Verreiben mit Äther einen amorphen Feststoff. Das Produkt wird isoliert und mehrfach mit Äther gewaschen. Man erhält das 7-(1,3-Thiazol-4-yl-glyoxylamido)-cephalosporansäure-β-S-oxyd als beigefarbenen Feststoff.

DC im Fließmittel wie im voranstehenden Beispiel: Rf 0,22.

IR in KBr: Lactam — CO bei 1788 $cm^{-1}$.

NMR: Thiazolprotonen bei δ 8,96 und 9,23 ppm ($(CD_3)_2SO$), jeweils Dubletts J = 2 Hz.

Herstellung der Ausgangsverbindungen

a) 1,3-Thiazol-4-yl-glyoxylsäureäthylester

Zu einer Lösung von 30 g Thioformamid in 100 ml Äthanol werden bei —10° eine Lösung von 89,2 g Bromacetglyoxylsäureäthylester in 100 ml Äthanol zugetropft. Nach 2 Stunden bei 0° wurde 2 Stunden ohne Kühlung gerührt, wobei die Reaktion nochmals mild exotherm wurde.

Anschließend wurde die Reaktionsmischung eingeengt, mit Methylenchlorid versetzt, das ausgefallene kristalline Produkt entfernt, die organische Phase eingeengt und im Vakuum destilliert.

Der 1,3-Thiazol-4-yl-glyoxylsäureäthylester geht als blaßgelbes Öl bei 1 mm bei 105—122° über.

Alternativ erhält man den 1,3-Thiazol-4-ylglyoxylester, indem man 17,1 g 1,3-Thiazol-4-ylessigsäureäthylester in 50 ml 1,2-Dichloräthan mit 10,5 g $SeO_2$ unter Rückfluß am Wasserabscheider erhitzt. Nach

Ende der Wasserabscheidung wird abgesaugt, der Rückstand mit 1,2-Dichloräthan gewaschen und die vereinigten Dichloräthanphasen eingeengt und der Rückstand destilliert.

b) 1,3-Thiazol-4-yl-glyoxylsäure

Zu 60 ml 2n NaOH werden 18,5 g des 1,3-Thiazol-4-yl-glyoxylesters unter Eiskühlung eingetragen, nach 10 Min. wird kurz erwärmt, erneut gekühlt, filtriert und das Filtrat mit konzentrierter HCl auf pH 3 gebracht. Die Lösung wird zur Trockne eingeengt, mit Aceton digeriert und isoliert. Der erhaltene Feststoff wird mit Methanol und Äther nachgewaschen. Man erhält die 1,3-Thiazol-4-yl-glyoxylsäure als cremefarbenen Feststoff vom Schmp. 185 – 190° (Zers.).

c) 2-(1,3-Thiazol-4-yl)-2-syn-methoximino-essigsäure

55 g 1,3-Thiazol-4-yl-glyoxylsäureäthylester werden bei Raumtemperatur in 500 ml Eisessig gelöst und unter Rühren mit einer Lösung von 23,4 g O-Methylhydroxylamin-hydrochlorid in 100 ml Wasser versetzt. Anschließend werden 30 g Natriumacetat hinzugefügt, die Reaktionsmischung 4 Stunden bei Raumtemperatur gerührt und schließlich in 2 l Wasser eingegossen. Man extrahiert mehrmals mit Chloroform, trocknet die vereinten organischen Phasen über $MgSO_4$ und engt zur Trockne ein.

Das zurückbleibende Öl wird in Toluol aufgenommen, über $Al_2O_3$-neutral filtriert und das Eluat erneut eingeengt. Man erhält den 2-(1,3-Thiazol-4-yl)-2-syn-methoximino-essigsäure-äthylester als Öl, das ohne weitere Reinigung in eine Lösung von 130 ml Äthanol und 130 ml 2n NaOH eingetragen wird. Nach kurzem Erwärmen auf dem Dampfbad wird auf pH 5 gestellt, zur Trockne eingeengt. Der Rückstand wird in 50 ml Wasser aufgenommen, mit 2n HCl der pH auf 1,5 gestellt, wobei das Produkt sich abscheidet und kristallisiert. Die Kristalle werden isoliert, mit Wasser gewaschen und getrocknet. Man erhält die 2-(1,3-Thiazol-4-yl)-2-syn-methoximino-essigsäure als farblose Kristalle vom Schmp. 156 – 158° (Zers.).

NMR: $[(CD_3)_2SO]$: 9,2 ppm (d, aromat. H), 8,1 ppm (d, aromat. H), 4,0 ppm (s, $O-CH_3$).

Alternativ erhält man die gleiche Verbindung, wenn man 20 g 2-(1,3-Thiazol-4-yl)-2-hydroximino-essigsäureäthylester (Synthese s. unter d) in 100 ml Aceton mit 27,6 g $K_2CO_3$ versetzt, zur Reaktionsmischung bei Raumtemperatur 13,8 g Dimethylsulfat hinzufügt und nach Ende der mild exothermen Reaktion 1 Stunde zum Sieden erhitzt. Die organische Phase wird nach dem Abkühlen isoliert, das Lösungsmittel abgedampft, der Rückstand in Chloroform aufgenommen und mehrfach mit Wasser gewaschen. Nach der Phasentrennung wird die organische Phase eingeengt und das zurückbleibende Öl des 2-(1,3-Thiazol-4-yl)-2-syn-methoximino-essigsäureäthylesters in der bereits beschriebenen Weise zur 2-(1,3-Thiazol-4-yl)-2-syn-methoximino-essigsäure verseift. Man erhält die Verbindung als farblose Kristalle vom Schmp. 156 – 159° (Zers.), die mit denen identisch sind, die durch die Umsetzung des 1,3-Thiazol-4-yl-glyoxylsäureesters mit O-Methylhydroxylamin hergestellt werden können.

d) 2-(1,3-Thiazol-4-yl)-2-syn-hydroximino-essigsäureäthylester

Eine Lösung von 18,5 g 1,3-Thiazol-4-yl-glyoxylsäureäthylester in 25 ml Eisessig wird mit einer Lösung von 9 g Natriumacetat in 10 ml Wasser und anschließend mit einer Lösung von 7,6 g Hydroxylamin-hydrochlorid in 10 ml Wasser versetzt.

Nach kurzem Stehen bei Raumtemperatur beginnt die Kristallabscheidung. Nach 2 Stunden wird die Reaktionsmischung gut gekühlt, die Kristalle abfiltriert, mit Wasser gewaschen und getrocknet und man erhält den 2-(1,3-Thiazol-4-yl)-2-syn-hydroxyiminoessigsäureäthylester in Form farbloser Kristalle vom Schmp. 175 – 178°.

e) 2-(1,3-Thiazol-4-yl)-2-syn-hydroxyiminoessigsäure

Die unter d erhaltenen Kristalle lassen sich mit 2n NaOH bei Raumtemperatur glatt verseifen. Man isoliert nach dem Ansäuern auf pH 2, Abfiltrieren, Nachwaschen mit Wasser und Trocknen die 2-(1,3-Thiazol-4-yl)-2-syn-hydroxyliminoessigsäure als cremefarbene Kristalle vom Schmp. 178 – 181° (Zers.).

f) 2-(1,3-Thiazol-4-yl)-2-syn-triphenylmethoximinoessigsäure

Eine Lösung von 6 g 2-(1,3-Thiazol-4-yl)-2-syn-hydroximino-essigsäureäthylester in 75 ml Methylenchlorid wird mit 3,3 g Triäthylamin und 8,35 g Triphenylchlormethan versetzt, 3 Stunden unter

Rückfluß gekocht, die Lösung gekühlt und mit Wasser gewaschen. Die organische Phase wird über $MgSO_4$ getrocknet, das Lösungsmittel abdestilliert und der Rückstand mit Äther versetzt. Das erhaltene Produkt wird isoliert, mit Äther gewaschen und getrocknet, wobei der 2-(1,3-Thiazol-4-yl)-2-syn-triphenylmethoximinoessigsäureäthylester als Feststoff vom Schmp. 130 – 134° isoliert wird.

Der erhaltene Ester wird in einer Mischung aus 110 ml 2n NaOH und 110 ml Äthanol 2 Stunden auf dem Dampfbad erhitzt, anschließend 16 Stunden bei Raumtemperatur belassen, gekühlt und mit 2n HCl auf pH 2 angesäuert. Dabei entstehen Kristalle, die abgesaugt und mit Wasser gewaschen und im Vakuum getrocknet werden. Man isoliert die 2-(1,3-Thiazol-4-yl)-2-syn-triphenylmethoximinoessigsäure vom Schmp. 160 – 163° (Zers.).

g) 2-(1,3-Thiazol-4-yl)-2-syn-hydroximino-essigsäureäthylester

Man löst bei –30° C 21,5 g 2-(2-Aminothiazol-4-yl)-2-syn-hydroximino-essigsäureäthylester in 180 ml unterphosphoriger Säure, setzt 35 ml Salpetersäure (d = 1,4) und 13,8 g Natriumnitrit in 25 ml Wasser langsam zu. Nach Beendigung der Stickstoffentwicklung läßt man auf Raumtemperatur kommen, neutralisiert mit Soda (pH = 4), verdünnt mit ca. 100 ml Wasser und extrahiert erschöpfend mit Äther. Die organische Phase hinterläßt beim Einengen die Titelverbindung als bräunliches Öl, das beim Stehen kristallisiert.

Schmp. 175 – 177°.

Die Verseifung zur Säure wurde nach der Vorschrift e) ausgeführt.

h) 2-(1,3-Thiazol-4-yl)-2-syn-methoximino-essigsäureäthylester

α) Man löst bei –10° C 22,9 g (0,1 Mol) 2-(2-Aminothiazol-4-yl)-2-syn-methoximino-essigsäureäthylester in 140 ml Phosphorsäure (d = 1,7), setzt unter Rühren 40 ml Salpetersäure (d = 1,4) und anschließend 13 g Natriumnitrit in 20 ml Wasser tropfenweise zu. Das Reaktionsgemisch tropft man bei –5° in ein Gemisch von 310 ml Unterphosphoriger Säure ($H_3PO_2$) und 14 g Cu-Pulver. Nachdem die Stickstoffentwicklung beendet ist (ca. 30 Minuten) neutralisiert man mit Soda und extrahiert das Reaktionsgemisch mehrmals mit Äther. Die getrocknete organische Phase hinterläßt beim Eindampfen den 2-Syn-methoximino-2-thiazol-4-yl-essigsäureäthylester als gelbliches Öl, das ohne weitere Reinigung durch alkalische Verseifung zur Säure verwandelt wurde.

Rf-Wert ($SiO_2$/Äther) 0,57.

NMR: [$(CD_3)_2SO$] 9,2 ppm (S, aromat. H), 8,1 ppm (s, aromat. H), 4,1 – 4,5 ppm (q, $-CH_2$-Ester), 4,0 ppm (s, $-OCH_3$), 1,2 – 1,4 ppm (t, $CH_3$-Ester).

β) 2-(2-Brom-thiazol-4-yl)-2-syn-methoximino-essigsäureäthylester

Man löst bei –10° C 22,9 g (0,1 Mol) 2-(2-Amino-thiazol-4-yl)-2-syn-methoximino-essigsäureäthylester in 140 ml Phosphorsäure (d = 1,7), setzt unter Rühren 40 ml Salpetersäure (d = 1,4) und anschließend 13 g Natriumnitrit in 20 ccm Wasser tropfenweise zu. Dieses Reaktionsgemisch tropft man bei –50° C in ein Gemisch von 310 ml 48%iger Bromwasserstoffsäure und 14 g Cu-Pulver. Nach Beendigung der Stickstoffentwicklung neutralisiert man mit Soda, verdünnt mit Wasser und extrahiert viermal mit je 500 ml Äther. Die getrocknete organische Phase ergibt nach Entfernen des Lösungsmittels und Vakuumdestillation (0,4 mm/114° C) den 2-(2-Brom-thiazol-4-yl)-2-syn-methoximino-essigsäureäthylester.

Charakteristisch für die syn-Konfiguration der Methoximinogruppe ist die Lage des aromatischen Protons des Thiazolringes bei 7,5 ppm ($CDCl_3$) und die der $-OCH_3$-Gruppe bei 4,0 ppm. Die Signale dieser Gruppen in der anti-Verbindung liegen entsprechend bei 8,1 ppm und 4,1 ppm.

5,8 g (0,02 Mol) 2-(2-Brom-thiazol-4-yl)-2-syn-methoximino-essigsäureäthylester löst man in 75 ml Äthanol, setzt 3 g Diäthylamin zu und hydriert mit Ra-Nickel bei Normaldruck. Nach etwa 1/2 Stunde ist die theoretische Menge Wasserstoff verbraucht. Der Katalysator wird vorsichtig abgesaugt, das Lösungsmittel entfernt und durch Behandeln mit Äther der 2-(1,3-Thiazol-4-yl)-2-syn-methoximino-essigsäureäthylester vom Diäthylamin-hydrobromid abgetrennt.

**Patentansprüche für die Vertragsstaaten: BE, CH, DE, FR, GB, IT, NL, SE**

1. Cephalosporinderivate der allgemeinen Formel I

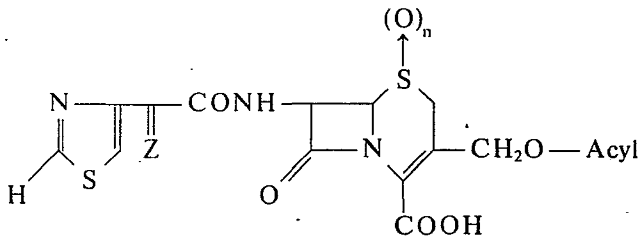

(I)

in der n=0 oder 1 bedeutet, Acyl für aliphatisches Acyl mit 1–5 C-Atomen steht, Z Sauerstoff oder =NOR bedeutet, worin R für Wasserstoff, Alkyl oder Alkenyl mit bis zu 5 C-Atomen steht, sowie deren pharmakologisch verträgliche Salze und Ester.

2. Verfahren zur Herstellung von Cephemderivaten der allgemeinen Formel I gemäß Anspruch 1, dadurch gekennzeichnet, daß man Thiazolylcarbonsäuren der allgemeinen Formel III

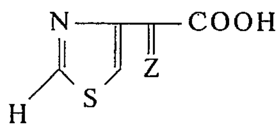

(III)

in der Z die im Anspruch 1 angegebene Bedeutung besitzt, worin R außerdem noch eine Schutzgruppe sein kann, in Form ihrer zur Amidbildung befähigten Derivate mit Cephemverbindungen der allgemeinen Formel II

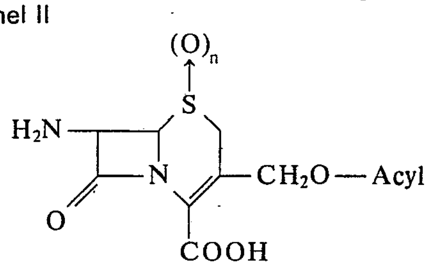

(II)

in der n und Acyl die im Anspruch 1 wiedergegebenen Bedeutungen besitzen, umsetzt und gegebenenfalls die Cephemderivate der Formel I mit n=0 zu Verbindungen der Formel I mit n=1 oxydiert und gegebenenfalls aus Z in der Bedeutung von =N–OR den Rest R abspaltet, soweit er für eine Schutzgruppe steht.

3. Gegen bakterielle Infektionen wirksame pharmazeutische Präparate, gekennzeichnet durch einen Gehalt an Cephemderivaten der allgemeinen Formel I.

4. Verfahren zur Herstellung von gegen bakterielle Infektionen wirksamen pharmazeutischen Präparaten, dadurch gekennzeichnet, daß ein Cephemderivat der allgemeinen Formel I gegebenenfalls mit pharmazeutisch üblichen Trägerstoffen oder Verdünnungsmitteln in eine pharmazeutisch geeignete Verabreichungsform gebracht wird.

**Patentanspruch für den Vertragsstaat: AT**

Verfahren zur Herstellung von Cephemderivaten der allgemeinen Formel I

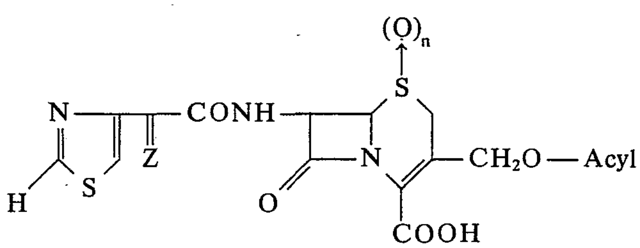

(I)

in der n=0 oder 1 bedeutet, Acyl für aliphatisches Acyl mit 1–5 C-Atomen steht, Z Sauerstoff oder =NOR bedeutet, worin R für Wasserstoff, Alkyl oder Alkenyl mit bis zu 5 C-Atomen steht, sowie deren pharmakologisch verträglichen Salzen und Estern, dadurch gekennzeichnet, daß man Thiazolylcarbonsäuren der allgemeinen Formel III

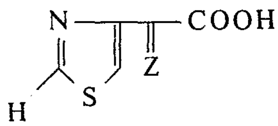

(III)

in der Z die oben angegebene Bedeutung besitzt, worin R außerdem noch eine Schutzgruppe sein kann, in Form ihrer zur Amidbildung befähigten Derivate mit Cephemverbindungen der allgemeinen Formel II nen Formel II

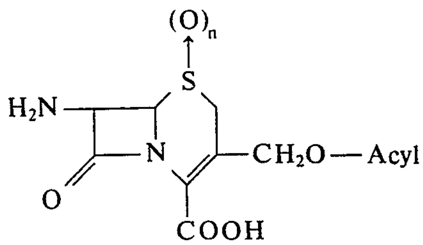

(II)

in der n und Acyl die oben wiedergegebenen Bedeutungen besitzen, umsetzt und gegebenenfalls die Cephemderivate der Formel I mit n=0 zu Verbindungen der Formel I mit n=1 oxydiert und gegebenenfalls aus Z in der Bedeutung von =N−OR den Rest R abspaltet, soweit er für eine Schutzgruppe steht.

**Claims for the Contracting States: BE, CH, DE, FR, GB, IT, NL, SE**

1. Cephalosporin derivatives of the general formula I

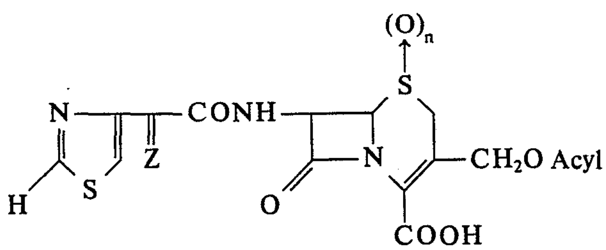

, (I)

in which n denotes zero or 1, Acyl denotes aliphatic acyl with 1 to 5 carbon atoms, Z denotes oxygen or =NOR in which R is hydrogen, alkyl or alkenyl with up to 5 carbon atoms and the pharmacologically acceptable salts and esters thereof.

2. Process for the manufacture of cephem derivatives of the general formula I as claimed in claim 1, which comprises reacting thiazolylcarboxylic acids of the general formula III

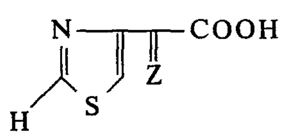

(III)

in which Z is as defined in claim 1, wherein R may further be a protective group, in the form of their activated derivatives capable of amide formation, with cephem compounds of the general formula II

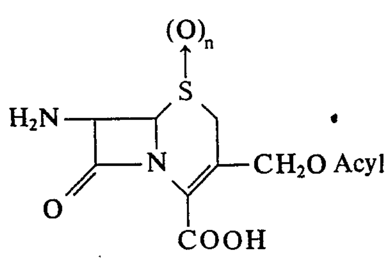

(II)

in which n and Acyl are as defined in claim 1, and optionally oxidizing the cephem derivatives of the formula I in which n denotes zero to give a compound of the formula I in which n is 1, and, in the case of Z denoting =N−OR, optionally splittung off the radical R when it denotes a protective group.

3. Pharmaceutical preparations having an action against bacterial infections, containing an amount of cephem derivatives of the general formula I.

4. Process for the manufacture of pharmaceutical preparations having an action against bacterial infections, which comprises bringing a cephem derivative of the general formula I, optionally with pharmaceutically customary excipients or diluents, into a pharmaceutically suitable form for administration.

**Claim for the Contracting State: AT**

Process for the preparation of cephem derivatives of the general formula I

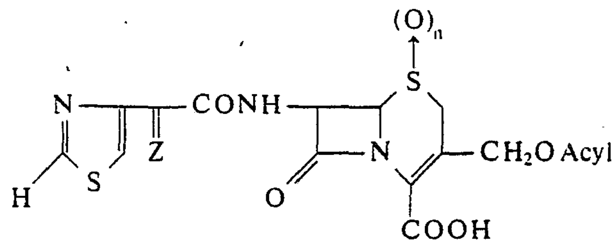

(I)

in which n denotes zero or 1, Acyl denotes aliphatic acyl with 1 to 5 carbon atoms, Z denotes oxygen or =NOR in which R is hydrogen, alkyl or alkenyl with up to 5 carbon atoms and the pharmacologically acceptable salts and esters thereof, which comprises reacting thiazolylcarboxylic acids of the general formula III

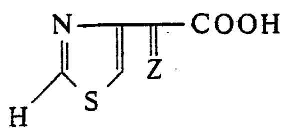

(III)

in which Z is as defined above, wherein R may further be a protective group, in the form of their activated derivatives capable of amide formation, with cephem compounds of the general formula II

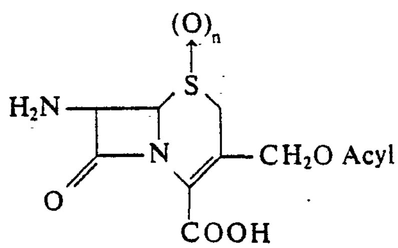

(II)

in which n and acyl are as defined above, and optionally oxidizing the cephem derivatives of the formula I in which n denotes zero to give compounds of the formula I in which n is 1, and, in the case of Z denoting =N−OR, optionally splitting off the radical R when it denotes a protective group.

**Revendications pour les Etats contractants: BE, CH, DE, FR, GB, IT, NL, SE**

1. Dérivés de la céphalosporine répondant à la formule générale I

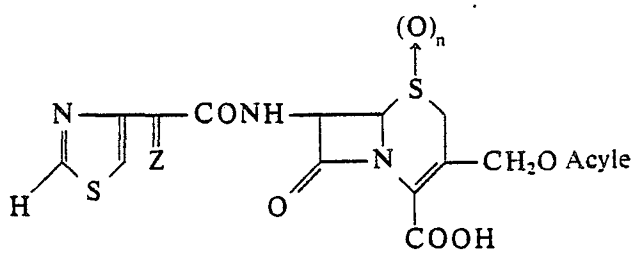

(I)

dans laquelle n est égal à 0 ou 1, Acyle désigne un groupe acyle aliphatique en $C_1$ à $C_5$, Z désigne l'oxygène ou =NOR, où R désigne l'hydrogène, un groupe alcoyle ou alcényle ayant jusqu'à 5 atomes de carbone, ainsi que leurs sels et esters pharmacologiquement acceptables.

2. Procédé de préparation de dérivés de la céphalosporine de formule générale I suivant la revendication 1, caractérisé en ce qu'on fait réagir des acides thiazolylcarboxyliques répondant à la formule générale III

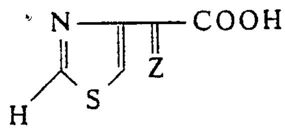

(III)

dans laquelle Z possède la signification indiquée ci-dessus dans la revendication 1, où R peut en outre être un groupe protecteur, sous la forme de leurs dérivés appropriés à la formation d'amide, avec des composés de la céphalosporine répondant à la formule générale II

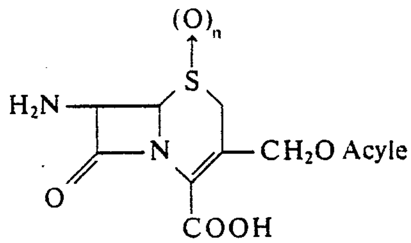

(II)

dans laquelle n et Acyle ont les significations indiquées ci-dessus dans la revendication 1, et on oxyde le cas échéant les dérivés de la céphalosporine répondant à la formule I avec $n=0$ en composés répondant à la formule I avec $n=1$, et on élimine le cas échéant de Z désignant $=N-OR$ le radical R, dans la mesure où il représente un groupe protecteur.

3. Préparations pharmaceutiques actives contre les infections bactériennes, caractérisées en ce qu'elles contiennent des dérivés de la céphalosporine répondant à la formule générale I.

4. Procédé de fabrication de préparations pharmaceutiques actives contre des infections bactériennes, caractérisé en ce qu'on amène un dérivé de la céphalosporine répondant à la formule générale I, le cas échéant avec des substances de support ou des diluants habituellement utilisés en pharmacie, sous une forme d'administration pharmaceutiquement appropriée.

**Revendication pour l'Etat contractant: AT**

Procédé de préparation de dérivés de la céphalosporine répondant à la formule générale I

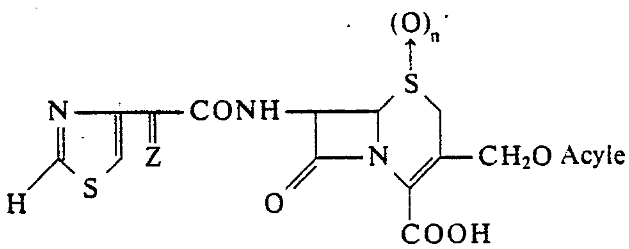

(I)

dans laquelle n est égal à 0 ou 1, Acyle désigne un groupe acyle aliphatique en $C_1$ à $C_5$, Z désigne l'oxygène ou $=NOR$, où R désigne l'hydrogène, un groupe alcoyle ou alcènyle ayant jusqu'à 5 atomes de carbone, ainsi que leurs sels et esters pharmacologiquement acceptables, caractérisé en ce qu'on fait réagir des acides thiazolylcarboxyliques répondant à la formule générale III

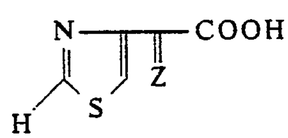

(III)

dans laquelle Z possède la signification indiquée ci-dessus dans la revendication 1, où R peut en outre être un groupe protecteur, sous la forme de leurs dérivés appropriés à la formation d'amide, avec des composés de la céphalosporine répondant à la formule générale II

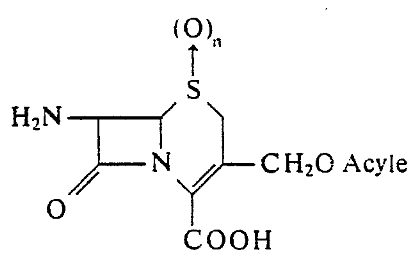

(II)

dans laquelle n et Acyle ont les significations indiquées ci-dessus dans la revendication, et on oxyde le cas échéant les dérivés de la céphalosporine répondant à la formule I avec n=0 en composés répondant à la formule I avec n=1, et on élimine le cas échéant de Z désignant =N—OR le radical R, dans la mesure où il représente un groupe protecteur.